# EUROPEAN PATENT SPECIFICATION

(11) **EP 3 735 931 B1**
(45) Date of publication and mention of the grant of the patent: **10.01.2024**
(21) Application number: 18898184.9
(22) Date of filing: 14.08.2018
(51) Int. Cl.: A61B 50/20, A61B 17/00, B25G 1/06, B25G 3/18, B25B 23/00

(54) **DUAL-USE HANDLE**
HANDGRIFF MIT DOPPELFUNKTION
POIGNÉE À DOUBLE USAGE

(30) Priority: 05.01.2018 CN 201810013119
(43) Date of publication of application: 11.11.2020
(73) Proprietor: Shanghai Reach Medical Instrument Co.,Ltd, Shanghai 201112 (CN); The Fourth Military Medical University of Chinese People's Liberation Army, Xi'an, Shaanxi 710032 (CN)
(72) Inventor: LUO, Zhuojing, Xian, Shaanxi 710032 (CN); HUANG, Xiaomin, Shanghai 201112 (CN)
(74) Representative: Valet Patent Services Limited
(86) International application number: PCT/CN2018/100316
(87) International publication number: WO 2019/134366

(56) References cited:
- CN-A- 102 507 069
- CN-A- 103 817 661
- CN-A- 108 210 081
- CN-B- 103 156 673
- CN-U- 204 160 394
- CN-U- 205 325 538
- CN-U- 205 325 538
- CN-U- 206 216 564
- DE-U1-202004 014 326
- DE-U1-202008 009 123
- US-A- 4 787 276
- US-A- 5 544 379
- US-A1- 2004 250 378
- US-A1- 2009 126 538
- US-A1- 2009 288 526
- US-A1- 2016 129 569

## Description

### Technical Field

The invention relates to the technical field of orthopedic medical devices and tools, in particular to a dual-purpose handle.

### Background

In medical operation, various forceps and threaded rod devices are required for assisting people in performing operations successfully, while the application of the forceps and rod devices needs assistance of handles. At present, handles used in the clinic are poor in interchangeability and single in functions; in this way, the types of the handles in a single operation are various, and the number is increased; the labor cost and the device acquisition cost are increased greatly, and moreover the acting force applied to the devices by hand are not liable to control; the adjustable range is small, and consequently the risk of damage to skeletons and relevant devices is increased.

CN 204 160 394 U describes an interchangeable multi-purpose handle comprising a rod part and a handle part, wherein at least one end of the rod part is formed with a wedge-shaped head, the middle section of the rod part is formed with a pair of symmetrical raised ribs, the handle part is axially provided with a channel matching the rod part, and radially provided with a through hole for the rod part to pass through, and at least one end of the handle part is formed with a clamping handle part. The interchangeable multi-purpose handle can be used as a driver when the rod part and the handle part are connected in the same straight line. The interchangeable multi-purpose handle can be used as a wrench when the rod part and the handle part are connected perpendicular to each other.

US 2009126538 A1 describes a body having one or more apertures to receive a variety of detachable utility inserts. The body being shaped with a spherical ball retainer member and/or a torsion bar for engaging a corresponding spherical indentation and notch formed on the utility insert. The body further includes a lever for locking and unlocking the utility insert from the body.

CN 103 156 673 B describes a rapid connection handle comprising a gripping handle and an insertion sleeve formed in the gripping handle, further comprising a movable sleeve provided in the insertion sleeve and slidably connected to the insertion sleeve, wherein a lateral through hole is formed on a side wall of the movable sleeve, a steel ball is provided in the above-mentioned lateral through hole, and the diameter of the steel ball is greater than the diameter of the lateral through hole. An inclined surface is formed on a side wall of the above-mentioned insertion sleeve. The inclined surface faces the bottom of the insertion sleeve and the position thereof corresponds to the position of the lateral through hole. The movable sleeve and the insertion sleeve are slidably connected by a spring.

CN 205 325 538 U describes an open end wrench comprising a handle, a connecting rod and a clamp with different specifications, wherein a mounting hole is provided on the handle, an inner end of the connecting rod is detachably inserted into the mounting hole. The mounting hole comprises a first mounting hole and a second mounting hole, wherein the first mounting hole is formed in a side wall of the handle, and the second mounting hole is formed at an end of the handle 12.

DE 2008 009123 U1 describes a screwdriver handle suitable for fixing a drill attachment, having openings with the diameter of 7 mm at both the front end and the lower side. Depending on the available space a user can decide whether to place the drill attachment at the front end of the handle or at right angles to the handle.

US 2004/250378 A1 describes a handle for a driving tool, the drive tool having a drive-tool handle. The handle has a body which is sized for hand gripping. The body has a longitudinal axis and a transverse axis. Formed in the body is a longitudinal bore, which has a cross section, extending from a first opening to a second opening in the body. A transverse bore extends from an opening in a side wall of the body and intersects the longitudinal bore.

US 4 787 276 A describes a hand tool for transferring torque having a handle, wherein tool implement blades may be alternately mounted upon the handle in three operative positions. A slot defined at one end of the handle allows installation of an implement having a forming head secured thereto such that the implement axis is parallel or lateral to the handle axis.

US 2016/129569 A1 describes a torque wrench comprising a solenoid driven release mechanism and a transceiver for wirelessly transmitting and receiving parameter set to detect the applied torque and to signal the operator that the torque set point has been reached.

US 2009/288526 A1 describes a hand tool with warning effect. A torque sensor, an operation torque detected by the torque sensor is transmitted to the processing unit. In the case that the value of the operation torque is within a set range, a processing unit shuts off the warning sound generator and a music player can play music for a user to listen to. Once the operation torque of the hand tool becomes greater than the set torque value, the processing unit turns on the warning sound generator to emit a warning signal to alert the user.

DE 2004 014326 U1 discloses an electronic wrench comprising a key body with a handle and at least one driving head, the handle being provided with electronics in a suitable position, the electronics comprising a voltmeter with a distance X from the centre of the driving head, and from which electronics can calculate a torque and display the torque value on a display.

### Summary of the Invention

The invention aims to provide a dual-purpose handle with the simple structure and easy operation, and the requirement of straight connection or T type connection between the dual-purpose handle and functional parts can be met.

In order to solve the above problems, the invention relates to a dual-purpose handle, comprising a body, and mounting holes are arranged in the body, and used to connect the functional parts. The mounting holes are arranged in the body axially and radially.

The mounting holes arranged in the body axially and radially enable the connection between the handle and the functional parts to switch between a straight type and a T type conveniently and efficiently. Different connection modes can output different torsional moments under the effect of the same operating force so that the control range is expanded.

As the further improvement of the invention, clamping stop devices which are fit with the functional parts are arranged in the mounting holes.

The clamping stop devices are arranged in the mounting holes. When the functional parts are connected with the handle, the functional parts can be fixed through the clamping stop devices axially, and prevented from being rotated in the body 1 cavity of the handle.

As the further improvement of the invention, the clamping stop devices are arranged as protrusions corresponding to grooves of the functional parts.

As the further improvement of the invention, the protrusions are elastomers.

When the functional parts are inserted and connected along the body cavity of the handle, the elastic protrusions arranged on the handle can locate the functional parts automatically. On the other hand, through pulling of the elastic protrusions arranged on the handle, the functional parts can be disengaged along the body cavity of the handle conveniently.

As the further improvement of the invention, the free ends of the protrusions are in smooth arc transition.

The smooth arc transition of the free ends of the protrusions enables the protrusions to slide in or out from grooves of the functional parts matched with the protrusions more smoothly.

As the further improvement of the invention, the protrusions and the body are arranged integrally and are in seamless connection.

Through the protrusions arranged with the body integrally, the handle has the advantages of low manufacturing cost, firm combination, long service life and the like.

As the further improvement of the invention, the mounting holes are arranged as through holes.

The axial hole and the radial hole of the handle are arranged as through holes; in this way, the handle is applicable for guide pins in different sizes to penetrate, and the handle is suitable for minimally invasive surgery; the application range of the handle is further expanded.

As the further improvement of the invention, anti-slip grooves or anti-slip protrusions are arranged on the body.

The anti-slip grooves or the anti-slip protrusions arranged on the body enable operating personnel to hold the handle in the using process more securely, and apply the acting force more conveniently.

As the further improvement of the invention, the body is made of ixef 1022 plastic.

According to claim 1, a strain gauge torque sensor is pasted on the surface of the body.

The strain gauge torque sensor is pasted on the surface of the body, and can be used to measure the torque applied to the functional parts by the operating personnel in real time; in this way, the risk is reduced greatly.

### Brief Description of the Drawings

In order to describe the embodiments of the invention or the technical scheme in the prior art more clearly, a brief introduction to the drawings required to use in the embodiment or the description of the prior art is made below, and obviously the drawings in the following description are only some embodiments of the invention; for those skilled in the art, on the premise that no creative work is done, other drawings can be further obtained according to these drawings.
Figure 1 is a structure diagram of the dual-purpose handle.
Figure 2 is a diagram of a usage mode of the dual-purpose handle in the first embodiment of the invention.
Figure 3 is a diagram of a usage mode of the dual-purpose handle in the second embodiment of the invention.
1-body; 11-mounting hole; 111-axial mounting hole; 112-radial mounting hole; 2-clamping stop device; 21-radial clamping stop device; 22-axial clamping stop device; 3-functional part.

### Detailed Description of the Preferred Embodiments

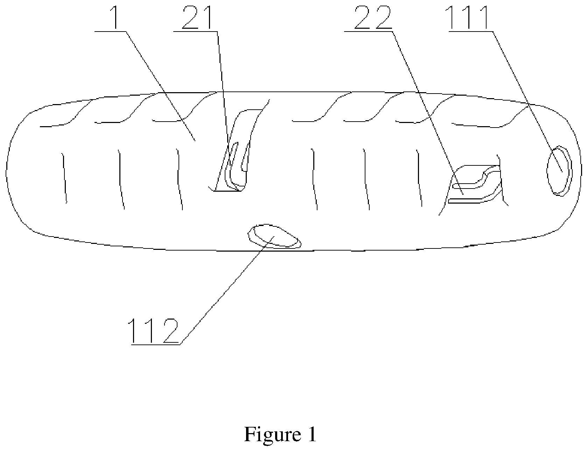

A detailed description of the dual-purpose handle of the invention is given below in combination with the embodiments of the invention:
In order to achieve the purposes of the invention, Figure 1 shows a structure diagram of the dual-purpose handle. The body 1 of the dual-purpose handle is made of ixef 1022 plastic. The anti-slip grooves or the anti-slip protrusions arranged on the body enable operating personnel to hold the handle in the using process more securely, and apply the acting force more conveniently. A radial mounting hole 112 and an axial mounting hole 111 are arranged in the body 1 of the dual-purpose handle radially and axially. The mounting holes are arranged as 1/4-inch international common holes so as to expand the application range of the handle as far as possible, and the types and number of the handle are reduced as far as possible. The functional parts 3 are connected with the body 1 through the above mounting holes (111 and 112). In addition, in order to enable the functional parts 3 to connect with the body 1 conveniently, quickly and reliably, the radial clamping stop device 21 and the axial clamping stop device 22 are arranged on the body 1 of the dual-purpose handle radially and axially. The clamping stop devices 21 and 22 are preferably designed as protrusions corresponding to the grooves of the functional parts, and can also be arranged in other forms meeting the clamping stop function. The protrusions arranged on the handle are elastomers, and the free ends of the elastomers are in smooth arc transition. When the functional parts 3 are inserted and connected along the body 1 cavity of the handle, the elastomers can locate the functional parts automatically; through pulling of the elastic protrusions arranged on the handle, the functional parts 3 can be disengaged along the body 1 cavity of the handle conveniently. The smooth arc transition of the free ends of the protrusions enables the protrusions to slide in or out from the grooves of the functional parts matched with the protrusions more smoothly. Certainly, in order to reduce the manufacturing cost, enable the protrusions to combine more securely and the service life to prolong, the protrusions and the body can be arranged integrally.

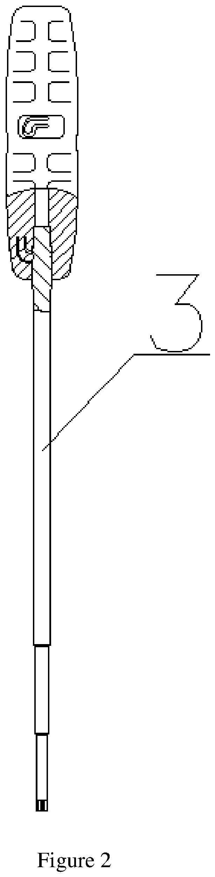

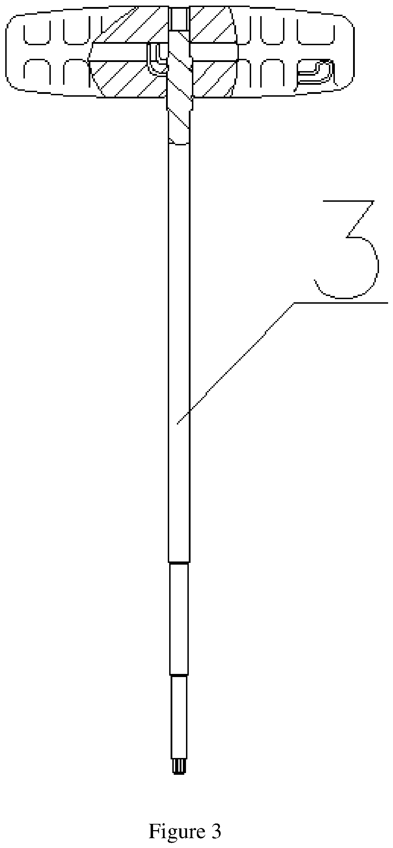

Figure 2 shows a diagram of a usage mode of the dual-purpose handle in the first embodiment of the invention. The functional parts 3 are in straight connection with the body through the axial mounting hole 111 on the body 1; Figure 3 shows a diagram of a usage mode of the dual-purpose handle in the second embodiment of the invention. The functional parts 3 are in T-type connection with the body through the radial mounting hole 112 on the body 1. The arms of the acting forces are different in the above two connection modes are different so that the controllable range of the torque is expanded. The straight connection can achieve small torque, while the T type connection is used to achieve large torque; in this way, the operability of the handle in the using process is improved. As the further improvement, the strain gauge torque sensor is pasted on the surface of the body, and shows data on a display screen in time so as to measure the torque applied to the functional parts by the operating personnel in real time; in this way, the operating process is more accurate and effective, and the risk is reduced greatly.

As the further improvement, the axial hole and the radial hole of the handle are arranged as through holes; in this way, the handle the handle can be suitable for minimally invasive surgery, and is applicable for guide pins to penetrate; the application range of the handle is further expanded.

The above description of the disclosed embodiments enables those skilled in the art to achieve or use the dual-purpose handle.

## Claims

1. A dual-purpose handle for orthopedic medical operation, comprising:
a body (1);
mounting holes (11) which are provided in the body (1) and are used for connecting to functional members, wherein
the mounting holes (11) are provided in the body (1) in both an axial direction and a radial direction, and **characterized in that** a strain gauge torque sensor is pasted on the surface of the body (1) for sending real-time data to a display screen so as to monitor the torque applied to the functional parts by operating personnel in real time.

2. The dual-purpose handle according to Claim 1, **characterized in that** clamping devices fitted with the functional members are arranged in the mounting holes (11).

3. The dual-purpose handle according to Claim 2, **characterized in that** the clamping devices are arranged as protrusions corresponding to grooves of the functional parts (3).

4. The dual-purpose handle according to Claim 3, **characterized in that** the protrusions are elastomers.

5. The dual-purpose handle according to Claim 4, **characterized in that** the free ends of the protrusions are in smooth arc transition.

6. The dual-purpose handle according to Claim 5, **characterized in that** the protrusions and the body (1) are arranged integrally and are in seamless connection.

7. The dual-purpose handle according to Claim 1, **characterized in that** the axial and radial holes of the handle are configured to be through holes.

8. The dual-purpose handle according to Claim 1, **characterized in that** anti-slip grooves or anti-slip protrusions are arranged on the body (1).

## Patentansprüche

1. Doppelzweckiger Griff für die orthopädische Chirurgie, umfassend einen Körper (1);
wobei am Körper (1) Befestigungslöcher (11) zur Herstellung von Verbindungen mit Funktionsteilen vorgesehen sind,
wobei sowohl in axialer als auch in radialer Richtung des Körpers (1) Befestigungslöcher (11) angeordnet sind,
**dadurch gekennzeichnet, dass**
ein Dehnungsmessstreifen-Drehmomentsensor an der Oberfläche des Körpers (1) geklebt ist, um Daten sofort an einen Bildschirm zu senden, so dass vom Bediener auf die Funktionsteile ausgeübte Drehmoment in Echtzeit überwacht wird.

2. Doppelzweckiger Griff nach Anspruch 1, **dadurch gekennzeichnet, dass** in die Befestigungslöcher (11) eine Verriegelungsvorrichtung versehen sind, die an die Funktionsteile angepasst ist.

3. Doppelzweckiger Griff nach Anspruch 2, **dadurch gekennzeichnet, dass** die Verriegelungsvorrichtung als ein der Nut des Funktionsteils (3) entsprechender Vorsprung ausgebildet ist.

4. Doppelzweckiger Griff nach Anspruch 3, **dadurch gekennzeichnet, dass** der Vorsprung ein Elastomer ist.

5. Doppelzweckiger Griff nach Anspruch 4, **dadurch gekennzeichnet, dass** das freie Ende des Vorsprungs einen glatten Bogenübergang bildet.

6. Doppelzweckiger Griff nach Anspruch 5, **dadurch gekennzeichnet, dass** der Vorsprung und der Körper (1) einstückig geformt und nahtlos verbunden sind.

7. Doppelzweckiger Griff nach Anspruch 1, **dadurch gekennzeichnet, dass** die Befestigungslöcher als Durchgangslöcher ausgebildet sind.

8. Doppelzweckiger Griff nach Anspruch 1, **dadurch gekennzeichnet, dass** an dem Körper (1) eine Anti-Rutsch-Rille oder ein Anti-Rutsch-Vorsprung vorgesehen ist.

## Revendications

1. Une manivelle à double usage destinée aux opérations orthopédiques, comprenant:
le corps (1) ;
Des trous de montage (11) sont prévus sur ledit corps (1), pour l'assemblage des pièces fonctionnelles, parmi lesquels
des trous de montage (11) sont aménagés dans les directions axiale et radiale dudit corps (1), la manivelle est **caractérisée en ce que** :
un capteur de couple à jauge de contrainte est collé sur la surface dudit corps (1), pour transmettre instantanément des données à l'écran d'affichage, permettant ainsi une surveillance en temps réel du couple appliqué par l'opérateur sur les pièces fonctionnelles.

2. Une manivelle à double usage selon la revendication 1, **caractérisée en ce qu'**un dispositif de blocage assorti à la pièce fonctionnelle est prévu dans ledit trou de montage (11).

3. Une manivelle à double usage selon la revendication 2, **caractérisée en ce que** ledit dispositif de blocage est prévu sous la forme d'une saillie correspondant au cran de ladite pièce fonctionnelle (3).

4. Une manivelle à double usage selon la revendication 3, **caractérisée en ce que** ladite saille est en élastomère.

5. Une manivelle à double usage selon la revendication 4, **caractérisée en ce que** le côté libre de ladite saille se profile en arc doux.

6. Une manivelle à double usage selon la revendication 5, **caractérisée en ce que** ladite saille est intégrée au corps (1) de manière monobloc sans joint.

7. Une manivelle à double usage selon la revendication 1, **caractérisée en ce que** lesdits trous de montage sont de type traversant.

8. Une manivelle à double usage selon la revendication 1, **caractérisée en ce que** sur ledit corps (1) sont prévues des rainures antidérapantes ou des saillies antidérapantes.
